# EUROPEAN PATENT SPECIFICATION

(11) **EP 0 208 672 B1**
(45) Date of publication and mention of the grant of the patent: **17.02.1993**
(21) Application number: 86870095.6
(22) Date of filing: 01.07.1986
(51) Int. Cl.: C12N 15/40, A61K 39/12

(54) **Vaccines and diagnostics derived from bovine diarrhea virus**
Vakzine und Diagnostika, die vom Bovine-Diarrhea-Virus stammen
Vaccins et compositions diagnostiques dérivés du virus de diarrhea bovin

(30) Priority: 08.07.1985 US 752981
(43) Date of publication of application: 14.01.1987
(73) Proprietor: REGION WALLONNE représentée par le Ministre des Technologies pour la Région wallonne dans ses attributions, B-1050 Bruxelles (BE); CHIRON CORPORATION, Emeryville, California 94608 (US)
(72) Inventor: Renard, André, B-4900 Angleur (BE); Dina, Dino, San Francisco California 94108 (US); Martial, Joseph, B-4050 Esneux (BE)
(74) Representative: Van Malderen, Michel

(56) References cited:
- AMERICAN JOURNAL OF VETERINARY RESEARCH, vol. 32, no. 7, July 1971 (Chikago, USA) A.L. FERNELIUS et al. "Evaluation of a Soluble Antigen Vaccine Prepared from Bovine Viral Diarrhea-Mucosal Disease Virus-Infected Cell Cultures" pages 1963-1979

## Description

### Technical Field

This invention relates to the field of vaccines and diagnostics for infectious diseases. Specifically, it relates to the disease syndrome caused by bovine diarrhea virus, and to vaccines, therapeutics, and diagnostics derived from the genomic sequence associated with the BDV virus.

### Background Art

Morbidity and mortality caused by bovine diarrhea virus (BDV) in dairy and beef herds is a worldwide unsolved economic problem. A subclinical form characterized by high morbidity and low mortality is endemic and is associated with diminished respiratory capacity, neonatal diarrhea, ulcerations in the digestive tract, immunodeficiency, and, in calf bearing bovines, abortion and teratogenicity. The disease is recognizable in calves, but adult carriers are difficult to identify.

An acute form of the disease results from infection of the fetus in the first trimester of pregnancy. The course of this form of the disease is insidious. The calves may survive the first infection, but those that do become immunotolerant, and excrete live viruses. They cannot survive a second infection. Since their capacity as carriers cannot be detected by titration of their sera, these animals are responsible for spreading of the disease from herd to herd.

BDV also infects hog populations. In hogs, it is important to distinguish animals as being infected by either BDV or hog cholera virus, since hog cholera is an economically important disease, while the bovine diarrhea infection is of transient significance, and could, for the most part, be ignored. Hogs infected with cholera must be slaughtered, and since present diagnostic methods in hogs cannot distinguish between these two types of infection, hogs which are, in fact, only infected with BDV must also be destroyed.

Present means of detection of BDV infection in calves are equally deficient, in that they rely on titration for antibodies in sera, which titration will fail to detect the immunotolerant calves. Thus, a diagnostic method is desired, but presently unavailable, which is capable both of detecting the presence of the virus in newborn animals with chronic infections, and in distinguishing between hog cholera virus and BDV infections. This could be accomplished either using antibodies with high affinity and specificity for the virus particles or using nucleic acid oligomeric probes capable of specific hybridization to the viral sequences.

Similarly, in addition to the need for improved diagnostics, there is, at present, no effective vaccine which is successful in preventing the spread of the disease caused by BDV. It is, of course, desirable that such a vaccine would confer long-term immunity, would not infect the fetus of the inoculated animal, and would have no undesirable side effects such as induction of immunotolerance to the virus, or depression of the immune system. These characteristics are difficult if not impossible to acquire in an attenuated or killed virus vaccine. Such vaccines, for the most part, constitute the present state of the art (Saurat, P., et al, "La Maladie des Muqueuses" (1972) pp. 229-251, L'Expansion scientific francaise Paris). Recently, Fernelius, A. L., et al, (Am J Vet Res (1971) 32:1963-1979) have reported a vaccine prepared from a high molecular weight soluble antigen obtained by density gradient centrifugation from BDV virus grown in embryonic bovine kidney cells.

The approaches used in the art for the detection of and protection against bovine viral diarrhea have been largely empirical and have not utilized refined knowledge of the nature of the vector causing the disease. The bovine diarrhea virus has, however, been classified, along with hog cholera and border disease viruses as a pestivirus which is a member of the family Togaviridae (Porterfield, J. S., "The Togavirions. Biology, Structure, Replication" Schlesinger. W., Ed. (1980), Academic Press, pp. 17-24).

By analogy to other togaviruses, these viruses should contain a capsid protein and two or three membrane glycoproteins (Horzinek, M.C., Non-arthrpodborne Togaviruses (1981), Academic Press, London. Epitopes which are capable of raising antibodies associated with neutralization and protection against infection are expected to be contained in the membrane proteins (e.g., see Boere, W., et al, J Virol (1984) 52:572-582). The pestiviruses are also characterized by soluble antigens that are approximately 80 kD proteins. A 76 kD protein from BDV has, in fact, been used as an experimental vaccine (Fernelius, A.L., et al, supra).

### Disclosure of the Invention

The invention is as described in the appended claims 1 to 21.

The invention provides cDNA copies of the entire bovine diarrhea virus RNA genomic sequence. This makes available the entire repertoire of peptides synthesized by the virus, and makes possible the preparation of proteins which contain epitopes effective and specific in generating desired antibodies and, in providing cells suitable for production of monoclonal antibodies. The primary structure of the genome also provides the necessary information to construct oligomeric sequences useful as diagnostic probes.

The protein products are thus able to serve as vaccines to protect animals subject to infection by this virus from subsequent illness. The accessibility of the entire genome provides opportunities for production of effective proteins, such as major virion components and individual virion subunits which would be unavailable using "native" production techniques, i.e., from viral infection of tissue cultured cells.

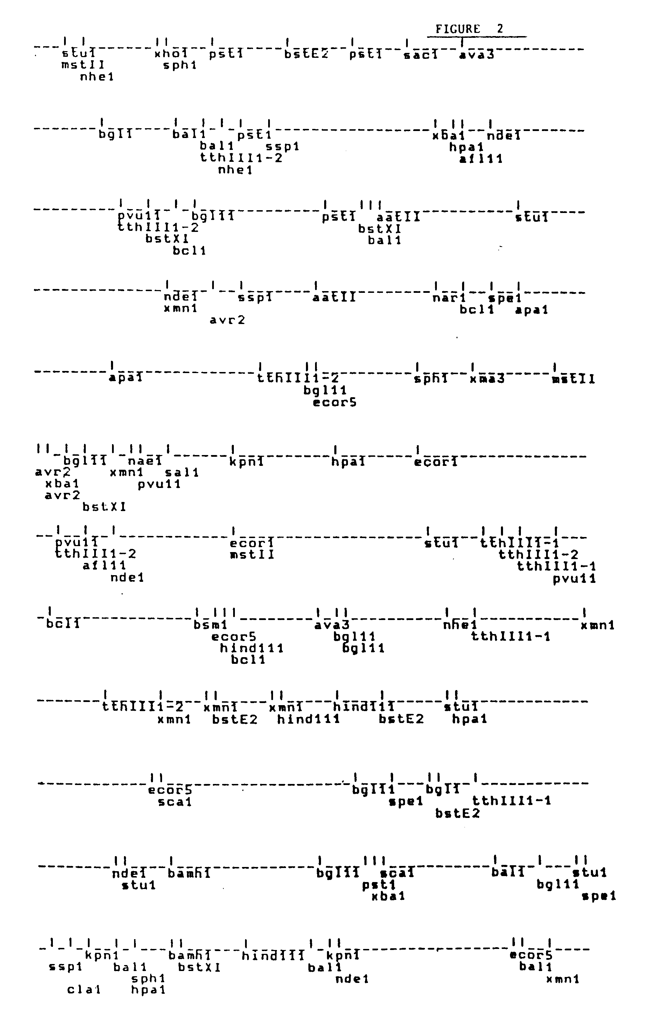

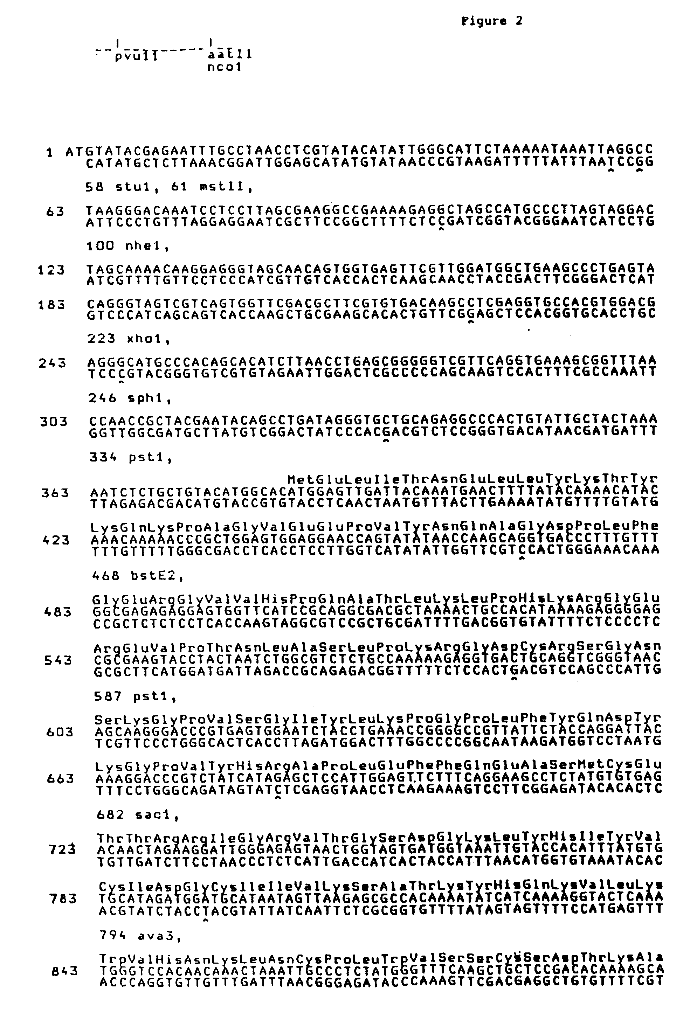

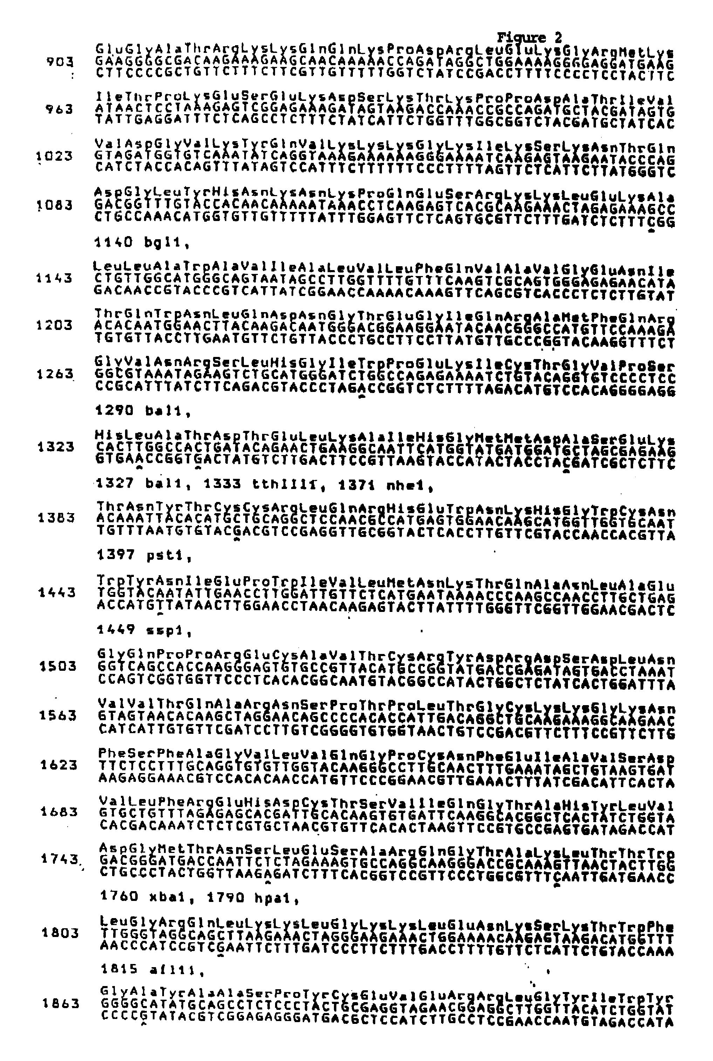

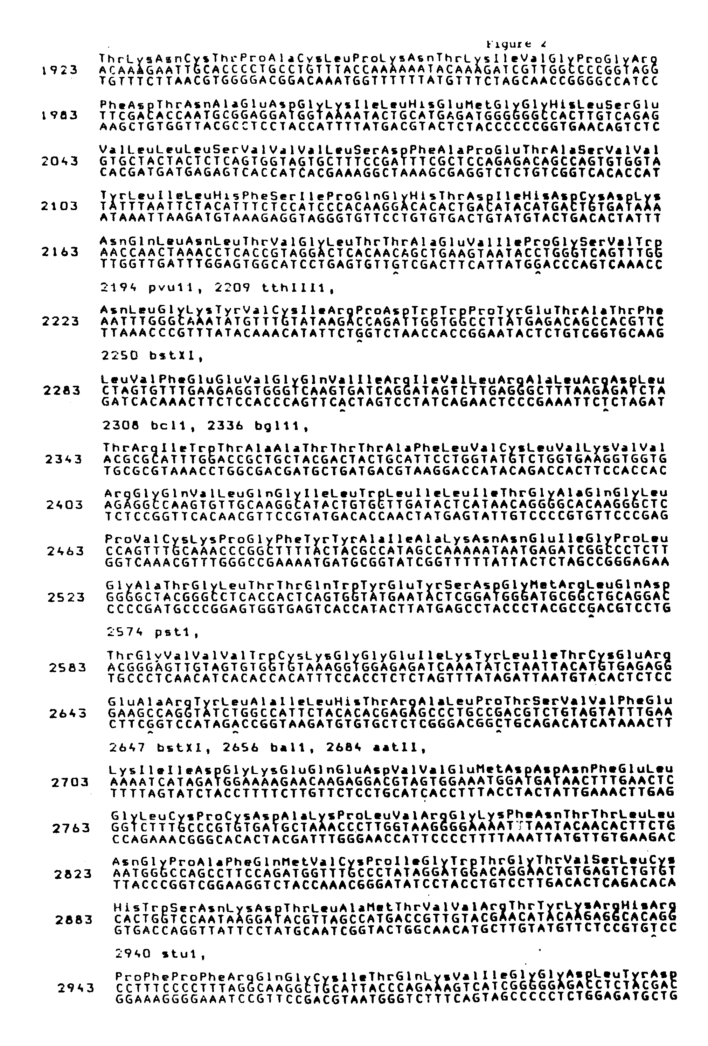

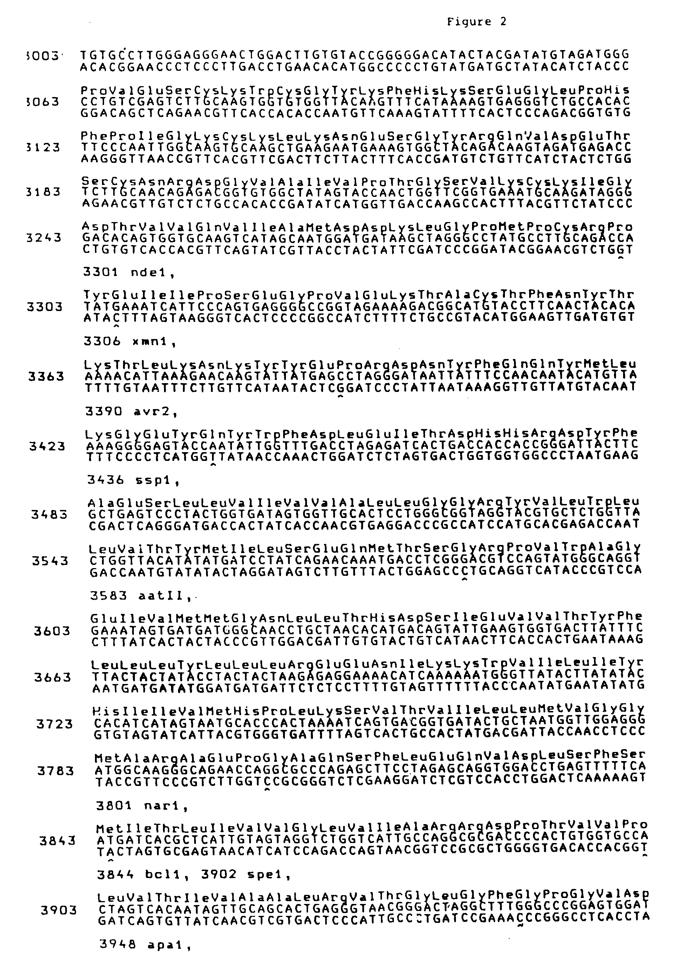

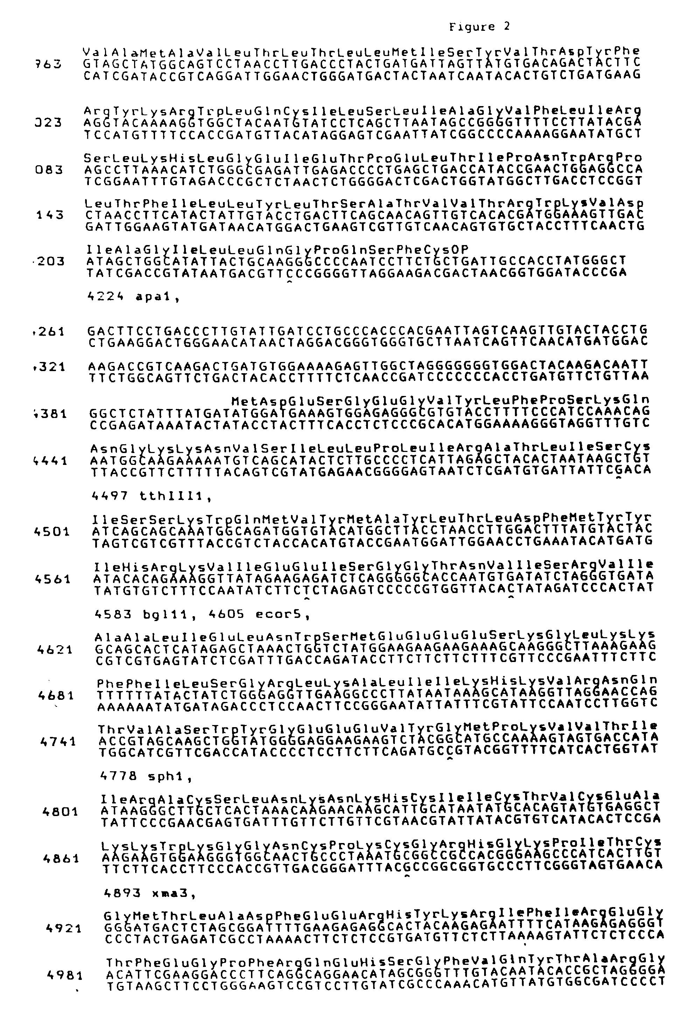

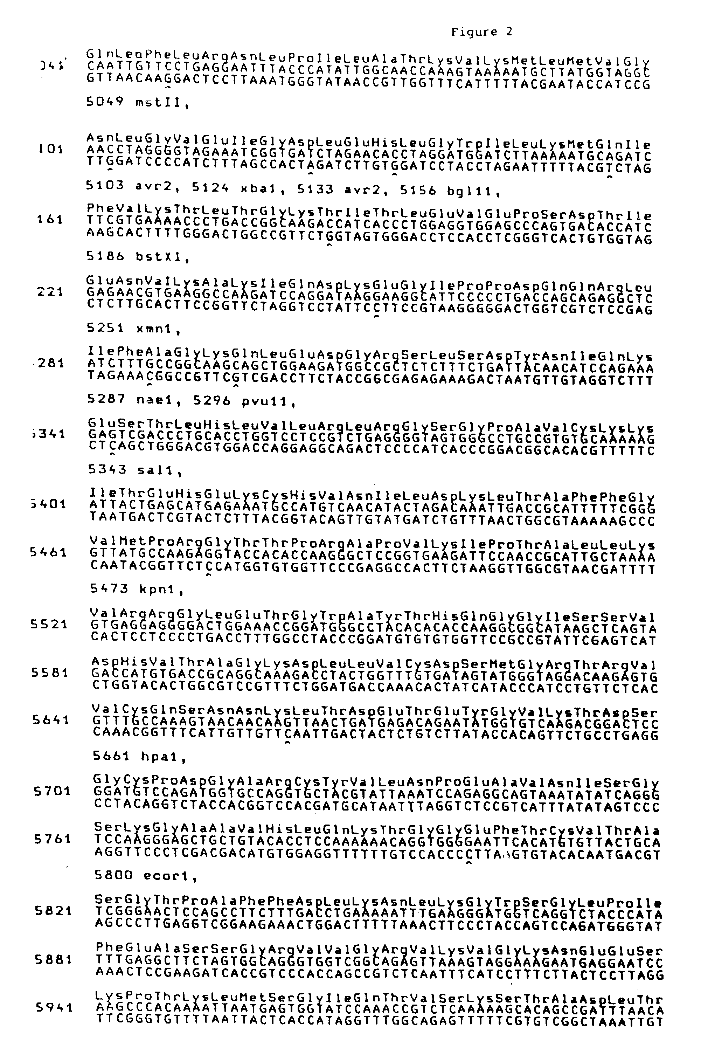

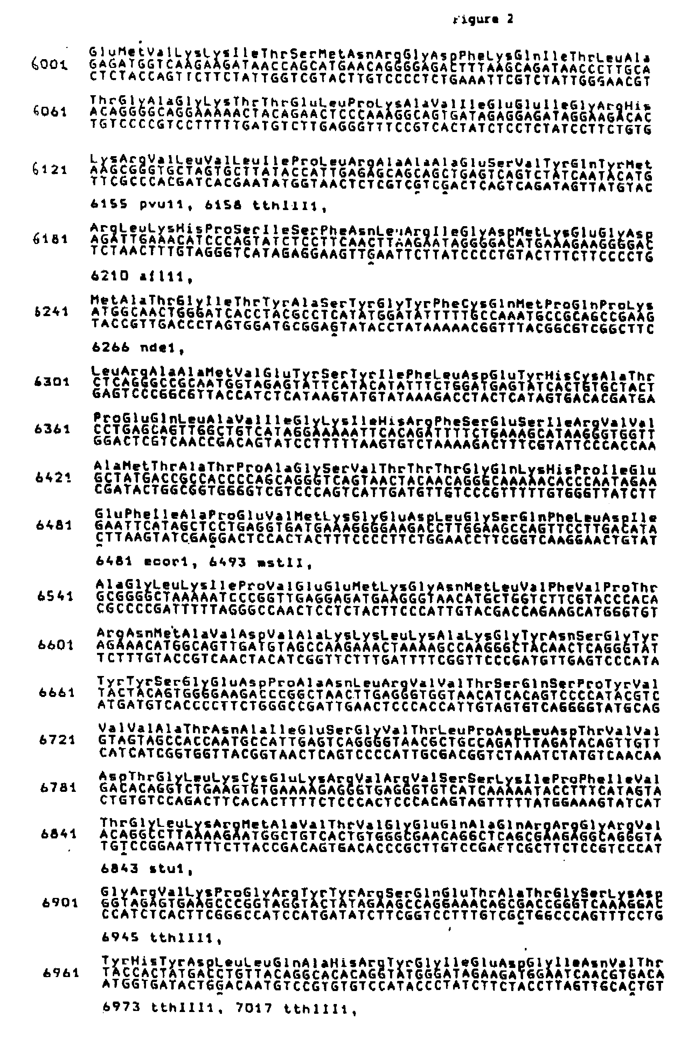

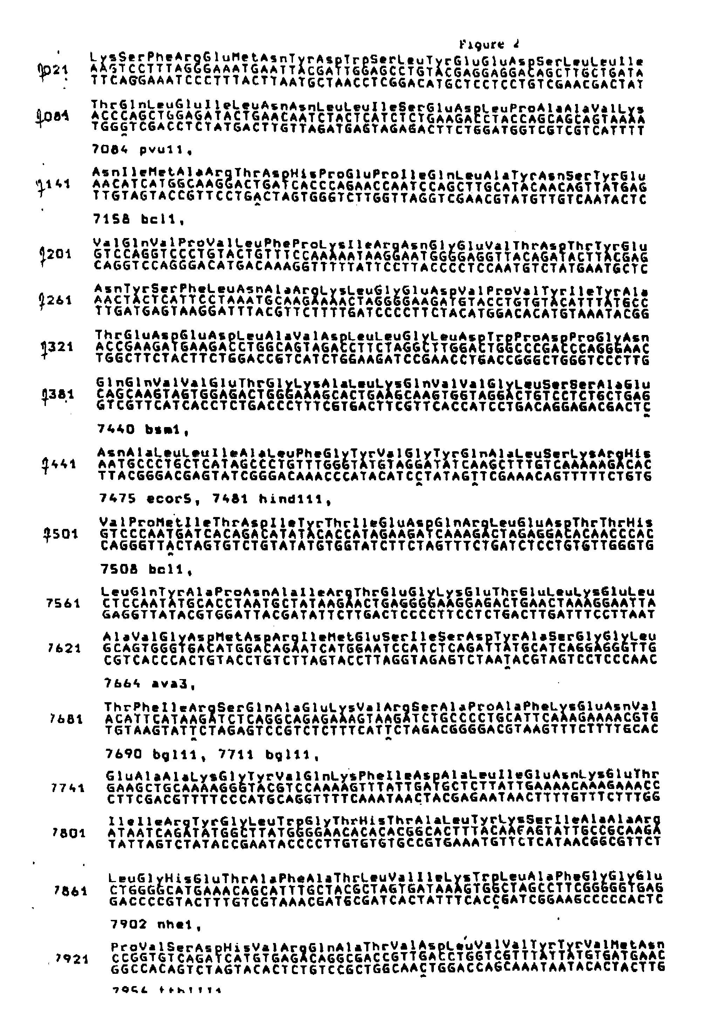

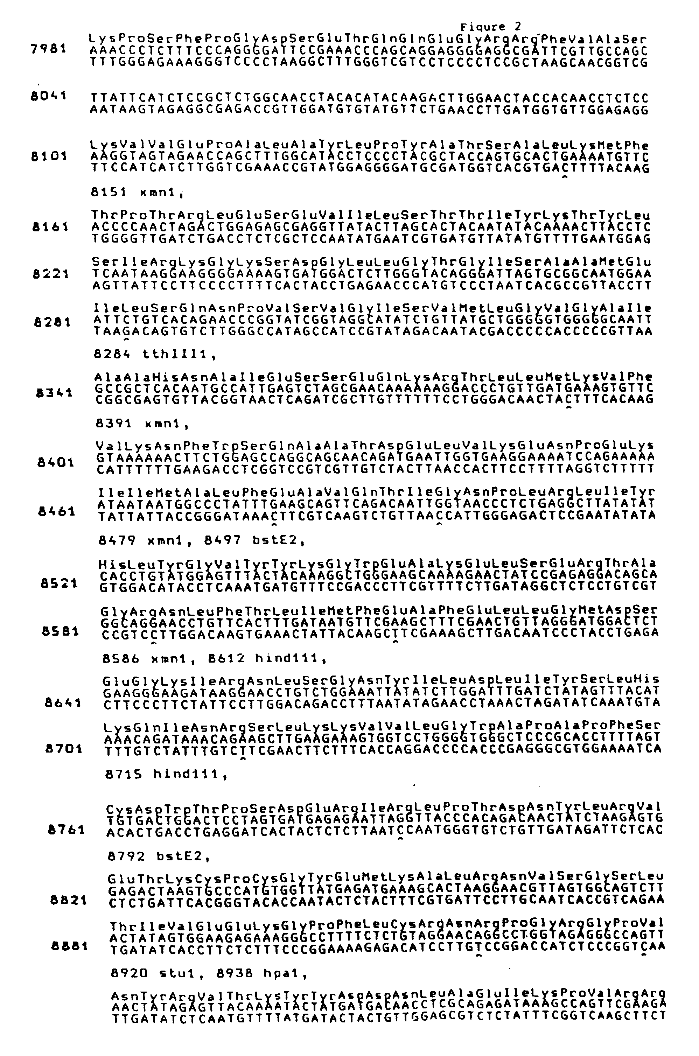

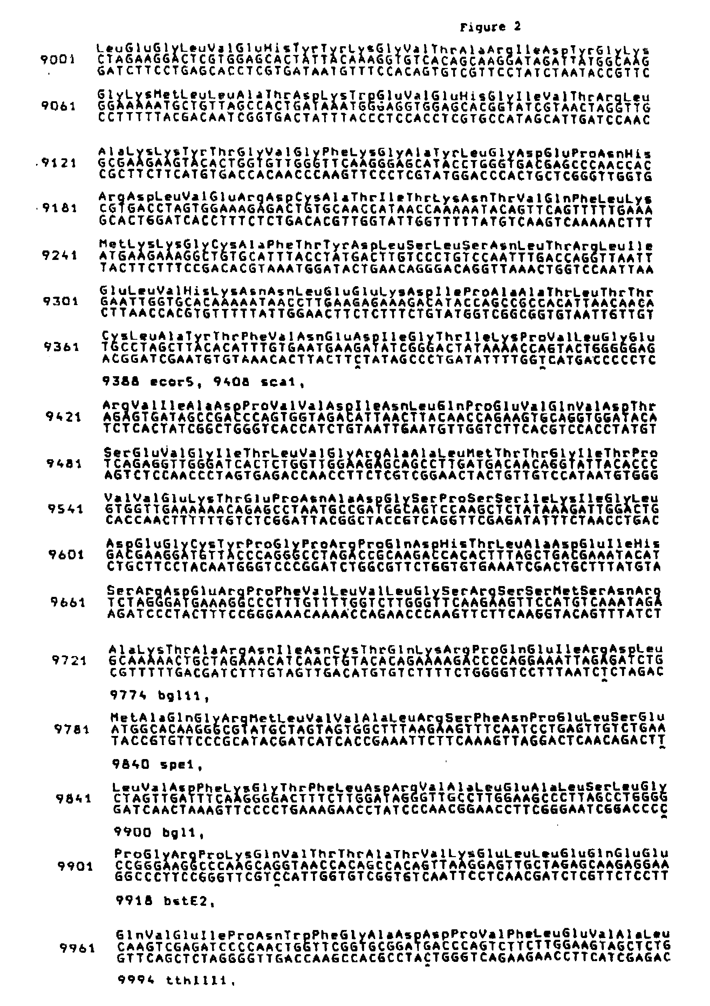

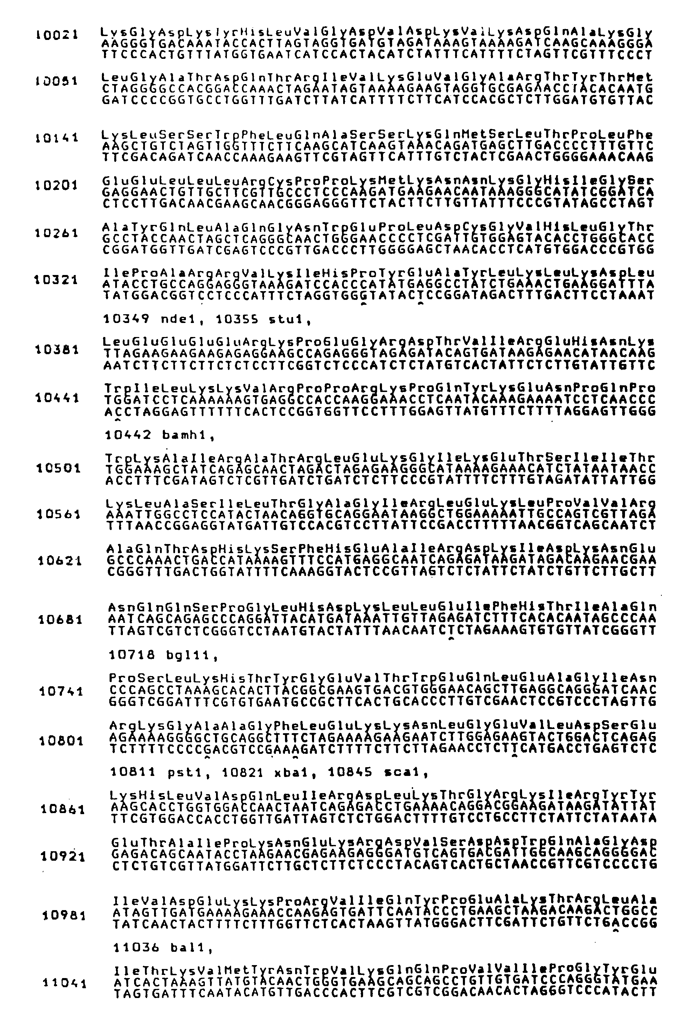

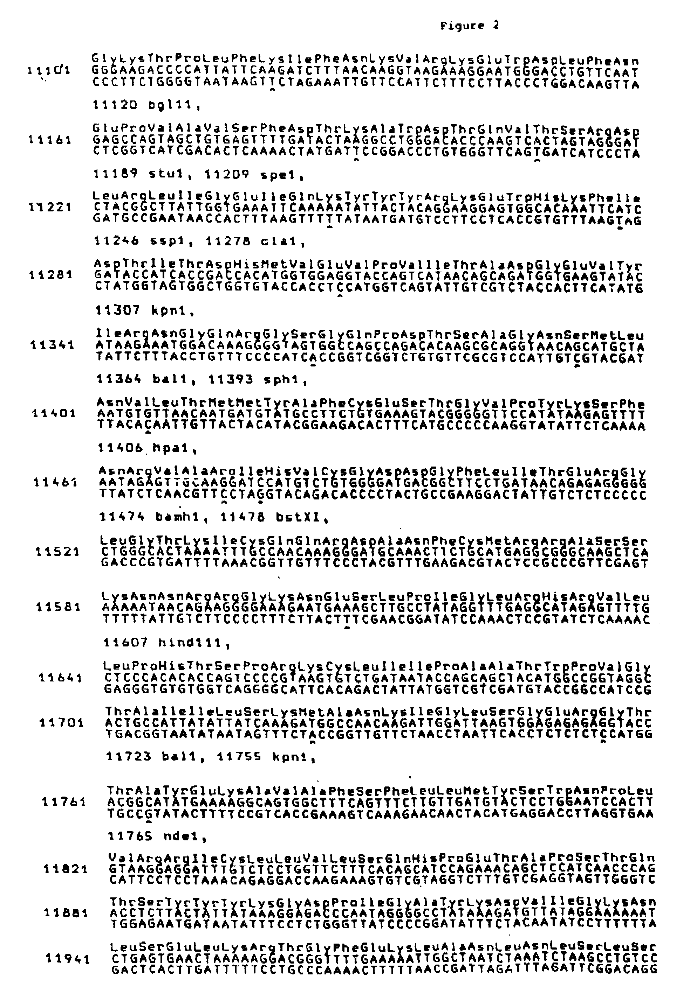

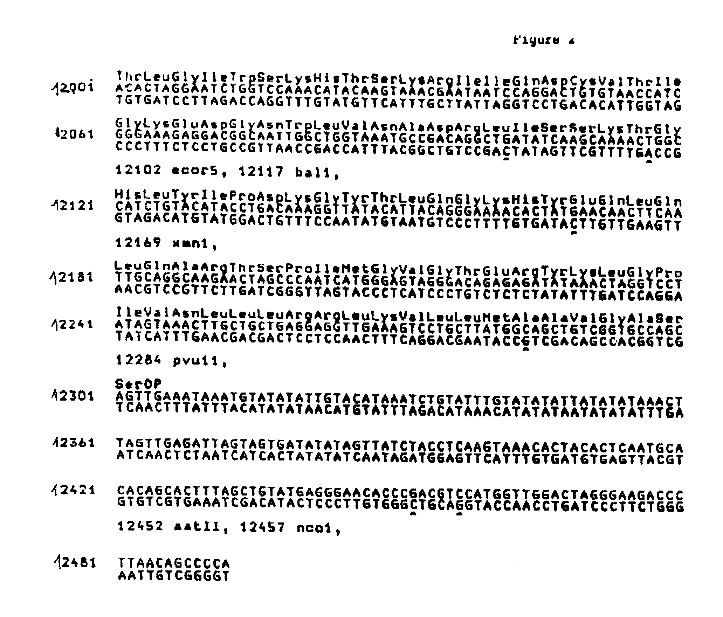

Accordingly, in one aspect, the invention relates to a nucleotide sequence substantially identical with that representing the entire genome of BDV as shown in Figure 2. Other aspects of the invention concern DNA or RNA sequences derived from portions of the genome, said sequences not necessarily representing contiguous portions. These are useful both as diagnostic probes and as coding sequences for desired proteins.

Other aspects of the invention include expression systems for the foregoing DNA derived from BDV which are effective in expressing this DNA in suitable heterologous hosts, including procaryotes, yeast, and mammalian cells. Live viral vectors, such as vaccinia, can also be used as carriers, and permit expression of the desired antigens along with the carriers' proteins in infected cells. Also included in the invention are hosts transformed with these expression systems and the proteins thus produced. The proteins produced in this way, or chemically synthesized to correspond to the deduced sequence, may be used as vaccines either alone, or in conjunction with carrier proteins which enhance their immunogenicity. In addition, the proteins may be used, either alone or conjugated with carrier, to elicit production of antibodies which are useful in diagnosis of carriers of the disease or in other immunoassays related to BDV.

The invention also relates to methods for preparing these polypeptide vaccines and immunoglobulins, and to methods of using the materials thus prepared.

### Brief Description of the Drawings

Figure 1 shows the map of overlapping segments of cDNA which, together, make up the entire BDV genomic sequence and cDNA fragments used to construct E. coli expression vectors.

Figure 2 shows the complete nucleotide sequence for the BDV genome. The cDNA contains the identical sequence, except, of course, that T will be substituted for U. The deduced amino acid sequence, based on the open reading frame, and confirmed by expression of segments is also shown.

### Modes of Carrying Out the Invention

### A. Definitions

As used herein, a nucleotide sequence "substantially identical" to the exemplified BDV genome refers to a sequence which retains the essential properties of the exemplified polynucleotide. A specific, but non-limiting example of such substantial equivalence would be represented by a sequence which encodes the identical or substantially identical amino acid sequence, but, which, because of codon degeneracy, utilizes different specific codons. Nucleotide changes are, indeed, often desirable to create or delete restriction sites, provide processing sites, or to alter the amino acid sequence in ways which do not adversely affect functionality. "Nucleotide sequence" refers both to a ribonucleotide and a deoxyribonucleotide sequence and includes the positive sense strand, as shown, and the negative sense strand as well.

A DNA sequence "derived from" the nucleotide sequence which comprises the genome of BDV refers to a DNA sequence which is comprised of a region of the genomic nucleotide sequence, or a combination of regions of that sequence. These regions are, of course, not necessarily physically derived from the nucleotide sequence of the gene, but refer to polynucleotides generated in whatever manner which have the same or "substantially identical" sequence of bases as that in the region(s) from which the polynucleotide is derived. For example, typical DNA sequences "derived from" the BDV genome include fragments encoding specific epitopes, fragments encoding portions of the viral polypeptide, sequences encoding the capsid proteins, sequences encoding deleted virions, and sequences encoding other useful viral genes.

"Recombinant host cells", "host cells", "cells", "cell lines", "cell cultures", and other such terms denoting microorganisms or higher eucaryotic cell lines cultured as unicellular entities, are used interchangeably, and refer to cells which can be, or have been, used as recipients for recombinant vector or other transfer DNA, and include the progeny of the original cell transfected. It is understood that the progeny of a single parental cell may not necessarily be completely identical in morphology or in genomic or total DNA complement as the original parent, due to accidental or deliberate mutation. Progeny of the parental cell which are sufficiently similar to the parent to be characterized by the relevant property, such as the presence of a nucleotide sequence encoding a desired peptide, are included in the progeny intended by this definition, and are covered by the above terms.

"Control sequence" refers to DNA sequences which are necessary to effect the expression of coding sequences to which they are ligated. The nature of such control sequences differs depending on the host organism: in procaryotes, generally such control sequences include a regulatory region promoter and ribosome binding site and termination signals; in eucaryotes, generally, such control sequences include promoters, terminators, and, in some instances, transcriptional enhancers. The term "control sequences" is intended to include, at a minimum, all components whose presence is necessary for expression, and may also include additional components whose presence is advantageous.

"Operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences.

### B. General Description

At the center of the present invention is the provision of a nucleotide sequence containing the entire genome of bovine diarrhea virus. The availability of this complete polynucleotide permits the design and production of oligomeric probes for diagnosis, of vaccines effective against BDV, and of proteins useful in production of neutralizing antibodies. Sequencing information available from the genome allows the amino acid sequence of the polypeptide to be deduced, and locations of favorable epitopes surmised. Further, once the desired sequences are chosen, appropriate fragments of the genome can be obtained and expressed independently, thus providing desired polypeptides. Short polypeptide fragments may also be chemically synthesized and linked to carrier proteins for use as immunogens. Recombinantly expressed polypeptides may be provided under conditions offering a favorable environment for processing into, for example, conjugation with cellular or artificial membranes which could thus bear the epitopic sites without the disadvantages of using an infectious virus. Mammalian and yeast cells provide suitable environments for such expression. In addition, the epitopes may be produced linked to a particle forming protein.

The above proteins produced may, themselves be used as vaccines, or may be used to induce immunocompetent B cells in hosts, which B cells can then be used to produce hybridomas that secrete antibodies useful in passive immunotherapy and diagnosis.

### B.1. Nucleotide Sequence of the BDV Genome

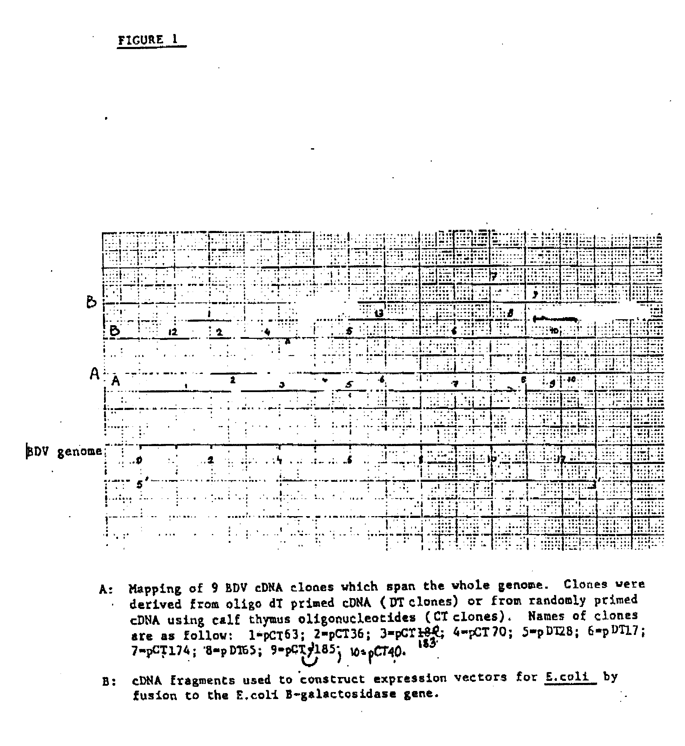

The genomic sequence of BDV was obtained from cDNA clones representing overlapping sections of the entire viral RNA genome (Figure 1). The viral RNA was isolated from virus grown on bovine embryonic kidney cells. The viral RNA was fractionated on sucrose gradients, and those fractions containing RNA of sufficient length to contain the intact genome were pooled, ethanol precipitated, and used to prepare a cDNA library. cDNA inserts were screened initially using a (+/-) system. Positive hybridizations were against RNA isolated from virus after lysis of infected cells, negative hybridizations were against RNA isolated from uninfected cells. One insert having the proper +/-response was then used as a reference clone to map the remainder of the library. Several colonies hybridizing to the positive insert were used to obtain additional portions of the viral genome from the cDNA library using "walking" techniques. Ten cDNA clones were obtained representing overlapping portions of the viral genome, as shown in Figure 1, and were subjected to restriction mapping and sequencing. The entire genomic sequence was deduced from these ten cDNA inserts, and is shown in Figure 2.

The illustrated DNA sequence and portions thereof are useful directly as diagnostic tools for detecting the presence of BDV in infected animals. These are particularly useful in distinguishing BDV infections from hog cholera virus. Methods to employ DNA hybridization in diagnosing disease have been disclosed in U.S. Patent No. 4,358,535 to Falkow. As set forth therein, biological samples may be used directly in obtaining Southern blots using suitable probes. Since the BDV genome is different from that of hog cholera virus, specific portions of the BDV sequence may be used to detect the presence of corresponding complementary sequences in biological samples from subjects suspected of harboring the infection.

### B.2. Preparation of Viral Polypeptide Fragments in E. coli

The availability of the entire genomic sequence permits construction of expression vectors encoding presumptively antigenically active regions of the virion proteins. Fragments encoding the desired proteins are obtained from the cDNA clones using conventional restriction digestion and ligated into a series of vectors containing polylinker sites in all possible reading frames to generate fusion proteins at the C-terminal end of β-galactosidase. Eleven portions of the BDV genome were expressed as β-gal fusions in E. coli using this approach, as outlined in Figure 1. These portions were obtained by restriction cleavage and/or ligation of the ten original clones, or the original cloned sequences were used directly. The fusion proteins thus produced may be immunogenic.

### B.3. Preparation of Antigenic Polypeptides and Conjugation with Carrier

Peptide regions representing epitopes can be synthesized using chemical or recombinant methods, and provided with, for example, cysteine residues at the C-terminus which provide means for linking the peptides to neutral carrier proteins. A number of techniques for obtaining such linkage are known in the art, including the formation of disulfide linkages using common reagents such as N-succinimidyl-3-(2-pyridyldithio)propionate (SPDP) and succinimidyl-4-(N-maleimido-methyl)cyclohexane-1-carboxylate (SMCC) obtained from Pierce Company. Rockford, Illinois. These reagents create a disulfide linkage between themselves and peptide cysteine residues in one protein and an amide linkage through the ε-amino on a lysine, or other free amino group in the other. A variety of such disulfide/amide-forming agents are known. See, for example. Immun Rev (1982) 62:185. Other bifunctional coupling agents form a thioether rather than a disulfide linkage. Many of these thioether-forming agents are commercially available and include reactive esters of 6-maleimidocaproic acid, 2-bromoacetic acid, 2-iodoacetic acid, 4-(N-maleimido-methyl) cyclohexane-1-carboxylic acid, and the like. The carboxyl groups can be activated by combining them with succinimide or 1-hydroxy-2-nitro-4-sulfonic acid, sodium salt. The foregoing list is not meant to be exhaustive, and modifications of the named compounds can clearly be used.

Any carrier may be used which does not itself induce the production of antibodies harmful to the subject, such as the various serum albumins, tetanus toxoids, or keyhole limpet hemocyanin (KLH).

The conjugates, when injected into suitable subjects, result in the production of antisera which contain immunoglobulins specifically reactive against not only these conjugates, but also against fusion proteins carrying the analogous portions of the sequence, and against whole BDV.

### B.4. Preparation of Mammalian Cell Membranes Containing BDV Epitopes

Portions of the cDNA library comprising the BDV genome were also ligated into expression vectors compatible with mammalian recombinant host cells; in the illustration below, into a mammalian/bacterial shuttle vector containing a linker sequence downstream of the SV40 early promoter, which is followed by the polyA sequence also derived from SV40. Alternate vectors to this particular host vector, pSV7d, could, of course, also be used. The mammalian-compatible vectors containing the coding sequences for the desired polypeptides are then transformed into suitable mammalian cells for expression of the sequences and, in the case of surface glycoproteins, transport of the produced protein to the membrane. The cells are ultimately harvested and used as whole cells in the formulation of vaccines, or the membranes are disrupted and portions of the membranes used correspondingly, or the proteins purified and formulated into vaccines.

### B.5. Preparation of Hybrid Particle Immunogens Containing BDV Epitopes

The immunogenicity of the epitopes of BDV may also be enhanced by preparing them in mammalian or yeast systems fused with particle-forming proteins such as that associated with hepatitis B virus (HBV) surface antigen (HBsAg). Constructs wherein a BDV epitope is linked directly to the particle-forming protein coding sequences produce hybrids which are immunogenic with respect to the BDV epitope, as well as to HBV epitopes.

Hepatitis B surface antigen has been shown to be formed and assembled in S. cerevisiae (Valenzuela et al, Nature (1982) 298:344-350. The formation of such particles has been shown to enhance the immunogenicity of the monomer subunit. The particles can also be formed from constructs which contain the presurface (pre-S) region in addition to the mature surface antigen. The pre-S region encodes an immunodominant HBV epitope and these proteins are expressed in yeast (Neurath et al, Science (1984) 224:392-394). Expression of constructs encoding pre-S region fused to particle forming protein are disclosed in European Patent Application 0 174 444. Expression of coding sequences for hybrid particles containing HBsAg and a heterologous epitope are disclosed in U.S. 4,722,840. These constructs may also be expressed in mammalian cells such as Chinese hamster ovary cells using an SV40-dihydrofolate reductase vector (Michelle et al, Int Symp on Viral Hepatitis (1984)).

In addition, portions of the particle-forming protein coding sequence per se may be replaced with codons for an BDV epitope. In this replacement, regions which are not required to mediate the aggregation of units to form immunogenic particles in yeast or mammals can be deleted, thus eliminating additional hepatitis B antigenic sites from competition with the BDV epitope.

### B.6. Vaccinia Carrier

Large, wide host range virus carriers have also been used in formulating vaccines by integrating the epitopic regions of the desired immunogen into the carrier viral genome. Vaccinia virus, in particular, has been used for this purpose. For example, Smith, G.L., et al, Proc Natl Acad Sci (USA) (1983) 80:7155-7159, disclose the integration of the hemagglutinin gene from influenza virus into the vaccinia genome and use of the resulting recombinant virus as a vaccine. Similarly, Panicali, D., et al, ibid (1982) 79:4927-4931, cloned the thymidine kinase gene from Herpes simplex virus into vaccinia. The availability of the BDV genome of the invention offers similar opportunities. The recombination is generally done by co-infecting cells both with vaccinia virus and with a chimeric plasmid carrying the desired coding sequence under the control of the transcriptional regulatory signals and RNA start site from the vaccinia virus gene adjacent to a translational start site/foreign protein coding sequence. During infection the similarity in the flanking DNA sequences of the foreign DNA sequences to those in vaccinia causes integration of the desired portion of the chimeric plasmid into the vaccinia genome. The resulting recombinant vaccinia can be harvested from the infected cells and used in the formulation of a vaccine. Vaccinia virus has an extremely large (120 x 10⁶ dalton) genome, and may be very easily grown in culture. Hence, the production of large amounts of inexpensive immunogenic vaccine is readily possible.

### B.7. Preparation of Vaccines

Preparation of vaccines which contain peptide sequences as active ingredients is also well understood in the art. Typically, such vaccines are prepared as injectables, either as liquid solutions or suspensions; solid forms suitable for solution in, or suspension in, liquid prior to injection may also be prepared. The preparation may also be emulsified or the protein encapsulated in liposomes. The active immunogenic ingredient is often mixed with excipients which are pharmaceutically acceptable and compatible with the active ingredient. Suitable excipients are, for example, water, saline, dextrose, glycerol, ethanol, or the like and combinations thereof. In addition, if desired, the vaccine may contain minor amounts of auxiliary substances such as wetting or emulsifying agents, pH buffering agents, or adjuvants which enhance the effectiveness of the vaccine. The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkaline glycols or triglycerides; such suppositories may be formed from mixtures containing the active ingredient in the range of 0.5% to 10%, preferably 1%-2%. Oral formulations include such normally employed excipitents as, for example, pharmaceutical grades of manitol, lactose, starch magnesium stearate, sodium saccharine, cellulose, magnesium carbonate and the like. These compositions take the form of solutions, suspensions, tablets, pills, capsules, sustained release formulations or powders and contain 10%-95% of active ingredient, preferably 25%-70%.

The proteins may be formulated into the vaccine as neutral or salt forms. Pharmaceutically acceptable salts, include the acid addition salts (formed with the free amino groups of the peptide) and which are formed with inorganic acids such as, for example, hydrochloric or phosphoric acids, or such organic acids as acetic, oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such amount as will be therapeutically effective and immunogenic. The quantity to be administered depends on the subject to be treated, capacity of the subject's immune system to synthesize antibodies, and the degree of protection desired. Precise amounts of active ingredient required to be administered depend on the judgment of the practititioner and are peculiar to each subject.

### B.8. Preparation of Mabs Against BDV Epitopes

The immunogenic proteins or immunoconjugates prepared as described above may be used to obtain peripheral blood lymphocytes and spleen cells in injected mammals to prepare hybridomas capable of secreting monoclonal antibodies directed against these epitopes. The resulting monoclonal antibodies are particularly useful in diagnosis, and, those which are neutralizing are useful in passive immunotherapy.

### C. General Methods

The general techniques used in extracting RNA from the virus, preparing and probing a cDNA library, sequencing clones, constructing expression vectors, transforming cells, and the like are known in the art and laboratory manuals are available describing these techniques. However, as a general guide, the following sets forth some sources currently available for such procedures, and for materials useful in carrying them out.

### C.1. Hosts and Expression Control Sequences

Both procaryotic and eucaryotic host cells may be used for expression of desired coding sequences when appropriate control sequences are used compatible with the designated host. Procaryotes are more useful for cloning; either procaryotes or eucaryotes may be used for expression. Among procaryotic hosts, E. coli is most frequently used, mostly for convenience. Expression control sequences for procaryotes include promoters, optionally containing operator portions, and ribosome binding sites. Transfer vectors compatible with procaryotic hosts are commonly derived from, for example, pBR322, a plasmid containing operons conferring ampicillin and tetracycline resistance, and the various pUC vectors, which also contain sequences conferring antibiotic resistance. The foregoing operons may be used as markers to obtain successful transformants by selection. Commonly used procaryotic control sequences include the β lactamase (penicillinase) and lactose promoter systems (Chang, et al, Nature (1977) 198:1056, the tryptophan (trp) promoter system (Goeddel, et al, Nucleic Acids Res (1980) 8:4057) and the λ derived P_{L} promoter and N gene ribosome binding site (Shimatake, et al, Nature (1981) 292:128). The foregoing systems are particularly compatible with E. coli; if desired other procaryotic hosts such as strains of Bacillus or Pseudomonas may be used, with corresponding control sequences.

Eucaryotic hosts include yeast and mammalian cell culture. Saccharomyces cerevisciae, or Baker's yeast and Saccharomyces carlsbergensis are the most commonly used yeast hosts, again because of convenience. Yeast compatible vectors carry markers which permit selection of successful transformants by conferring prototrophy to auxotrophic mutants or by conferring antibiotic resistance or resistance to heavy metals on wild-type strains. Yeast compatible vectors may employ the 2 micron origin of replication (Broach, J., et al, Meth Enz (1983) 101:307) the combination of CEN3 and ARS1, or other means for assuring replication, such as sequences which will result in incorporation of the appropriate fragment into the host cell genome. Control sequences for yeast vectors include promoters for the synthesis for glycolytic enzymes (Hess, et al, J Adv Enzyme Reg (1968) 7:149, Holland, et al, Biochemistry (1978) 17:4900), and the promoter for 3 phosphoglycerate kinase (Hitzeman, et al, J Biol Chem (1980) 255:2073). For yeast expression, terminators may also be included, such as those derived from the enolase gene (Holland, M. J., J Biol Chem (1981) 256:1385). Particularly useful control systems include those specifically described herein, which comprise the glyceraldehyde-3 phosphate dehydrogenase (GAPDH) promoter or alcohol dehydrogenase (ADH) regulatable promoter, terminators also derived from GAPDH, and, if secretion is desired, leader sequence from yeast alpha factor. These systems are described in detail in U.S. 4,876,197 and US 4,870,008

Mammalian cell lines available as hosts for expression include many immortalized cell lines available from the American Type Culture Collection, including HeLa cells, Chinese hamster ovary (CHO) cells, baby hamster kidney (BHK) cells, and a number of other cell lines. Suitable promoters for mammalian cells prominently include viral promoters such as that from Simian virus 40 (SV40) (Fiers, et al, Nature (1978) 273:113) or other viral promoters such as the Rous sarcoma virus (RSV) adenovirus, and bovine papiloma virus (BPV). Mammalian cells may also require terminator sequences. Vectors suitable for replication in mammalian cells may include viral replicons, or sequences which insure integration of the appropriate sequences into the host genome.

### C.2. Transformations

The transformation procedure used depends on the host to be transformed. Bacterial transformation generally employs treatment with calcium or rubidium chloride (Cohen, S. N., Proc Natl Acad Sci (USA) (1972) 69:2110, Maniatis, et al, Molecular Cloning: A Laboratory Manual (1982) Cold Spring Harbor Press, p. 254). Yeast transformations may be carried out using the method of Hinnen, A., et al, Proc Natl Acad Sci (USA) (1978) 75:1929-1933. Mammalian transformations are conducted using the calcium phosphate precipitation method of Graham and van der Eb, Virology (1978) 52:546, or the various modifications thereof.

### C.3. Vector Construction

Vector construction employs techniques which are by now quite well understood. Site-specific DNA cleavage is performed by treating with suitable restriction enzyme under conditions which generally are specified by the manufacturer of these commercially available enzymes (see, e.g., The New England Biolabs Product Catalog). In general, about 1 µg of plasmid or DNA sequence is cleaved by 1 unit enzyme in about 20 µl buffer solution for an incubation time of about 1-2 hr at about 37°C. After incubation with the restriction enzyme, protein is removed by phenol/chloroform extraction and the DNA recovered by reprecipitation with ethanol. The cleaved fragments may be separated using polyacrylamide or agarose gel electrophoresis techniques, according to the general procedures found in Methods in Enzymology (1980) 65:499-560.

Sticky ended cleavage fragments may be blunt ended using E. coli DNA polymerase I (Klenow) in the presence of the appropriate deoxynucleotide triphosphates (dNTPs) using incubation conditions appropriate to the polymerase. The polymerase digests protruding 3' single strands, but fills in 5' protruding ends, according to the dNTPs present in the mixture. Treatment with S1 nuclease may also be used, as this results in hydrolysis of any single stranded DNA portion.

Ligations are carried out using standard buffer and temperature conditions using T4 DNA ligase, and ATP; sticky end ligations require less ATP and less ligase than blunt end ligations. When vector fragments are used as part of a ligation mixture, the vector fragment is often treated with bacterial alkaline phosphatase (BAP) in order to remove the 5' phosphate and thus prevent religation of the vector; alternatively, restriction enzyme digestion of unwanted fragments can be used to prevent religation.

Ligation mixtures are transformed into suitable cloning hosts, such as E. coli, and successful transformants selected by, for example, antibiotic resistance, and screened for the correct construction.

### C.4. Construction of Desired DNA Sequences

Synthetic oligonucleotides may be prepared using an automated oligonucleotide synthesizer as described by Warner, B. D., et al, DNA (1984) 3:401-411. If desired, these synthetic strands may be kinased for labeling with ³²P by using an excess of polynucleotide kinase in the presence of labeled ATP, under standard kinasing conditions.

DNA sequences including those isolated from genomic or cDNA libraries may be modified by site directed mutagenesis, as described by Zoller, M, et al, Nucleic Acids Res (1982) 10:6487-6499. Briefly, the DNA to be modified is packaged into phage as a single stranded sequence, and converted to a double stranded DNA with DNA polymerase using, as a primer, a synthetic oligonucleotide complementary to the portion of the DNA to be modified, and having the desired modification included in its own sequence. The resulting double stranded DNA is transformed into a phage supporting host bacterium, and cultures of the transformed bacteria, which will contain replications of each strand of the phage, are plated in agar to obtain plaques. Theoretically 50% of the new plaques will contain phage having as a single strand the mutated form; 50% will have the original sequence. Replicates of the plaques are hybridized to kinased synthetic probe at temperatures and conditions which permit hybridization with the correct strand, but not with the unmodified sequence. The thus identified, desired, modified sequences are then recovered and cloned to serve as sources for the desired DNA.

### C.5. Hybridization with Probe

DNA libraries are probed using the procedure of Grunstein and Hogness (Proc Natl Acad Sci (USA) (1975) 73:3961). Briefly, in this procedure, the DNA to be probed is immobilized on nitrocellulose filters, denatured, and prehybridized with a buffer containing 0-50% formamide, 0.6 M NaCl, 60 mM sodium citrate, 0.02% (wt/v) each of bovine serum albumin, polyvinyl pyrollidine, and Ficoll, 50 mM sodium phosphate (pH 6.5), 1% glycine, and 100 µg/ml carrier denatured DNA. The percentage of formamide in the buffer, as well as the time and temperature conditions of the prehybridization and subsequent hybridization steps depends on the stringency desired. Oligomeric probes which require lower stringency conditions are generally used with low percentages of formamide, lower temperatures, and longer hybridization times. Probes containing more than 30 or 40 nucleotides such as those derived from cDNA or genomic sequences generally employ higher temperatures, e.g. about 40-42° and a high percentage, e.g. 50% formamide. Following prehybridization, this same buffer, now containing the ³²P kinased oligonucleotide probe, is added to obtain hybridization. Radioautography of the treated filters shows the location of the hybridized probe, and the corresponding locations on replica filters which have not been probed can then be used as the source of the desired DNA.

### C.6. Verification of Construction and Sequencing

For routine vector constructions, ligation mixtures are transformed into E. coli strain HB101 or other suitable host, and successful transformants selected by antibiotic resistance or other markers. Plasmids from the transformants are then prepared according to the method of Clewell, D. B., et al, Proc Natl Acad Sci (USA) (1969) 62:1159, usually following chloramphenicol amplification (Clewell, D. B., J Bacteriol (1972) 110:667). The isolated DNA is isolated and analyzed by restriction analysis, or sequenced by the dideoxy method of Sanger, F., et al, Proc Natl Acad Sci (USA) (1977) 74:5463, as further described by Messing, et al, Nucleic Acids Res (1981) 9:309, or by the method of Maxam, et al, Methods in Enzymology (1980) 65:499.

### D. Examples

The following examples are intended to illustrate but not limit the invention. The procedures set forth, for example, in ¶s D.1 and D.2 may, if desired, be repeated but need not be, as techniques are available for construction of the desired nucleotide sequences based on the information provided by the invention. Expression is exemplified in E. coli and in yeast, however other systems are available as set forth more fully in ¶C.1. Additional epitopes derived from the genomic structure may also be produced, and used to generate antibodies as set forth below.

### D.1. Preparation of cDNA

### D.1.a. Production of BVD Virus

Bovine Embryonic Kidney cells (BEKI) cells were grown in MEM (Earl's) containing 0.85 g/l NaHCO₃ and 10% of irradiated fetal calf serum. The biologically cloned Osloss strain of BVD virus was passaged 5 times through BEKI cells at a multiplicity of 0.1. Cytopathic effects, consisting of clustering of cells followed by vacuolation and then cell lysis, were readily observable from the first passage. Final titers (∼ 10⁸ pfu/ml) were obtained after recovery of virus by freezing and thawing of infected cells.

For the virus production, 175 cm² plastic flasks of subconfluent BEKI cells were used. The cells were washed 3 times with infection buffer (MEM (Earl's) + 2.2 g/l NaHCO₃, pH 7.6) and then were infected with 2 ml of BVD in infection buffer at a multiplicity of 0.05 pfu/cell. After 1 hr at 35°C, 18 ml of infection buffer was added and the cells were incubated for 4-5 days at 35°C, after which cytopathic effect (vacuolation followed by cells lysis) was greater than 80%. In a typical production, 150 flasks of cells were infected. The medium (about 3 liters) was collected and stored at 4°C. The remaining cells were scraped in 2 ml of infection buffer/flask, subjected to 3 cycles of freezing and thawing, and the final suspension was added to the infection medium. After a centrifugation at 10,000 g for 30 min, the supernatant was concentrated 10-fold by ultracentrifugation at 120,000 g for 4 hrs and 40 min at 4°C.

Infectious virus had a density of 1.12 g/ml as measured by isopicnic banding in sucrose density gradient, and appeared as 45-55 nm spherical particles by electron microscopy. The virus preparations were neutralized by anti-BVD antiserum from rabbits injected with virus or from bovines.

### D.1.b. Extraction and Purification of Viral RNA

RNA was isolated from the virus pellet by the CsCl/guanidinium thiocyanate method as described by Chirgwin, et al, Biochemistry (1979) 18:3294, and the purified RNA stored in 70% ethanol at -20°C. This RNA preparation contained a large amount of contaminating low molecular weight cellular RNA and intact viral RNA. Viral RNA was further purified by sucrose density gradient centrifugation as follows:

An aliquot containing an estimated amount of 5 µg of BVD-RNA was centrifuged at 10,000 g for 15 min at 4°C. The pellet was washed with 80% ethanol, denatured in 375 µl of 99% DMSO (99%), 5 mM Tris-HCl (pH 7.5) and incubated for 5 min at 37°C. After addition of 1.125 ml of 5 mM Tris HCl (pH 7.5), 1mM EDTA, 1% Sarkosyl, the solution was heated for 2 min at 70°C and quenched on ice. This solution was distributed on 5x15-30% sucrose gradients in 5 mM Tris HCl (pH 7.5), 10 mM EDTA, 0.1M NaCl, 1% Sarkosyl (in sterile siliconized Beckman SW40 tubes). A sixth gradient was loaded with 3' end labeled RNA as a marker (see below). After a centrifugation for 16 hrs at 19,000 rpm (20°C), the gradients were fractionated (1 ml fractions). The RNA from each fraction of the gradient corresponding to that containing marker-labeled RNA was precipitated with 2.5 volumes of ethanol in the presence of carrier yeast RNA (10 µg) and subjected to formaldehyde agarose gel electrophoresis, Lehrach, et al, Biochemistry (1977) 16:4743, to determine which fraction contained the BDV-RNA band. Fractions corresponding to those containing the BDV-RNA, were pooled from the parallel gradients and precipitated with 2.5 volumes of ethanol, washed with 80% ethanol and stored at -20°C in 70% ethanol.

The purified viral RNA was labeled with ³²P-pCp (3000 Ci/n mol) according to England, et al, Meth Enzymol (1980) 65:65-74, and analyzed by agarose gel electrophoresis in the presence of 2.2 M formaldehyde as described in Lehrach, et al, (supra). Fluorography was done with ³H-Enhancer (NEN) as recommended by the manufacturer.

The majority of the radioactivity was associated with low molecular weight RNA (less than 2 kb), but a small proportion was found in a high molecular band approximately 12.5 kb, identified as RNA by labeling properties with RNA ligase, its sensitivity to RNAse and alkali, and resistance to DNAse and proteinase K. In agreement with other reports on togaviruses of the flavivirus group, the BDV-RNA did not bind to oligo dT cellulose, showing either the absence of a polyA stretch at the 3' end, or that, if present, the polyA is extremely short. Control Sindbis virus RNA was properly retained by the same column.

These properties of the 12.5 kb band were identical with those shown by RNA extracted from BEKI cells, grown as follows:
BEKI cells were grown in 25 cm² plastic flasks, washed 3 times with infection buffer, and infected at multiplicities of 50-100 pfu/cell with 1 ml of BDV solution. After one hour at 35°C, 4 ml of infection buffer was added and the incubation was continued. After 12, 15, 18, 21 and 36 hrs (36 hr corresponds to a complete cycle of BDV replication), the newly synthesized RNA was labeled with ³H-uridine (100 µCi/dish). Uninfected cellular RNA harvested after 18 hrs of incubation was also analyzed. After 30 min of labeling, the cellular RNA was extracted using the CsCl/guanidinium thiocyanate method of Chirgwin et al, 1979 (supra). The pellet of RNA, obtained after ultracentrifugation through a 5.7 M CsCl cushion, was directly analyzed by formaldehyde agarose gel electrophoresis and gel was dried and fluorographed. In all the incubation times tested, a 12.5 kb band which is absent in the uninfected cells could be detected which has the same physico-chemical properties as shown by the RNA above.

### D.1.c. Preparation of cDNA

The viral RNA isolated from the virus in ¶D.1.b. was polyadenylated using the method of Sippel, Eur J Biochem (1973), 37:31-40. Briefly, the estimated amount of 0.7 µg of purified BVD RNA was incubated in 5 ml of 5 mM methylmercury hydroxide for 10 min at room temperature and incubated for 6 min at 37°C with 20 units of polyA polymerase (BRL) and 500 µCi of ³H-ATP (36 Ci/mmol, Amersham) in 50 µl of 50 mM HCl (pH 7.5), 10 mM MgCl₂, 2.5 mM MnCl₂, 0.3 M NaCl, 1.5 mM 2-mercaptoethanol and containing 2.5 µg of RNAse-free BSA and 5 units of human placental ribonuclease inhibitor (BRL). After phenol/chloroform extraction, the RNA was purified by chromatography on Sephadex G50 and precipitated with 2.5 volumes of ethanol. The polyA RNA was used to prepare probes and as a template for the cDNA library.

To make probes 1 µg of the polyA RNA was incubated for 10 min at room temperature in 5 µl of 10 mM methylmercury hydroxide and then 45 min at 37°C with 40 units of reverse transcriptase in 100 ml of 50 mM Tris HCl (pH 8.3), 10 mM MgCl₂, 1.5 mM 2-mercaptoethanol, 1 mM dATP, dGTP and dTTP, 10 µM dCTP, 0.2 mg/ml of actinomycin D, 5 units of human placental ribonuclease inhibitor, 500 µCi of alpha ³²P-dCTP (3000 Ci/mmole, Amersham) and 20 µg of oligonucleotides obtained by partial digestion with DNAse I of calf thymus DNA (random primers). After 15 and 30 min, ten more units of reverse transcriptase were added. After phenol/chloroform extration and Sephadex G50 column chromatography the RNA was hydrolyzed with 0.1 M NaOH (1 hr at 65°C) thus yielding single stranded cDNA strands. The solution was neutralized with 0.1 M acetic acid and added directly to the hybridization buffer.

For the cDNA library two separate cloning protocols involving dT (12-18) primers or random (calf thymus), DNA-derived oligonucleotide primers were used. RNA polyadenylated in vitro as described above was used. Approximately 1 µg polyadenylated RNA was incubated with 10 mM methylmercury hydroxide in a 10 µl volume for 10 min at room temperature, and excess reagent was titrated by adding 1 µl of a 3M 2-mercaptoethanol solution. This denatured polyA RNA was used immediately in the presence of 50 mM Tris pH 8.0, 1 mM dATP, dGTP, dCTP and dTTP, 2.5 µg/ml dT12-18 or the calf thymus random oligonucleotide primers, 10 mM MgCl₂, 10 µg/ml actinomycin D, 100 units of RNAse inhihitor (BRL) and 60 units of reverse transcriptase in a total volume of 100 µl.

The samples were diluted to 400 µl with a buffer containing 10 mM Tris pH 7.0, 100 mM NaCl, 10 mM EDTA and 0.2% SDS extracted with phenol/chloroform, freed of dNTPs by Sephadex G50 chromatography, and ethanol precipitated.

The precipitated mixture of RNA and cDNA hybrids (10 µl) were diluted into 50 ml of S1 buffer (500 mM NaCl, 50 mM Na acetate pH 4.5 and 1 mM ZnCl₂ and digested for 15 min at room temperature with 20 units of S1 nuclease. The reaction was stopped by diluting the sample to 500 ml with a buffer containing 50 mM NaCl, 10 mM EDTA and 50 mM Tris pH 7.0, and digestion was continued for 15 min at room temperature by adding 20 µg/ml of RNAse A. After phenol and chloroform extraction, the RNA:cDNA hybrids were concentrated by ethanol precipitation and fractionated on a Sepharose CL4B column prepared in a 1 ml plastic pipette. The excluded peak, containing molecules larger than 800 base-pairs, was pooled and ethanol precipitated to give 50 ng of hybrid for the dT primed, and 200 ng of hybrid for the random calf thymus fragment primed reactions.

Both samples were tailed for dC residues under conditions yielding 15-25 residues per DNA or RNA termini, and annealed to a dG tailed pBR322 vector linearized at the PstI site (NEN) at a vector concentration of 0.1 µg/ml. The annealed plasmids were transformed into E. coli HB101 to Amp^{R} to obtain the cDNA library.

### D.2. Screening of the cDNA Library

Screening employed a +/- method using labeled cDNAs prepared from RNA isolated from uninfected BEKI cells (-probe) and from RNA isolated from the virus obtained after complete lysis of the cells (+ probe). Colonies of the E. coli harbored cDNA library were grown, lysed on nitrocellulose filters (two replicas) and probed. The hybridization buffer used for + probe also contained an excess of cellular RNA isolated from uninfected BEKI cells (10 mg/ml). The colonies which gave a clear signal with the + probe and no response with the - probe were selected. By this method, 95 oligo dT-primed and 185 random primer primed clones were selected. The length of the inserts after PstI digestion varied from 400 to 4,000 base pairs. No full-length virus specific cDNA was obtained.

One of the clones, pDT28, with a 880 bp insert was selected for further analysis. This fragment from a PstI digest of plasmid DNA was purified by acrylamide gel electrophoresis, digested with DdeI and MboI and then labeled with the Klenow fragment of DNA polymerase I and the four ³²P dNTPs to yield 10⁶-10⁴ cpm/mg of insert. Labelled insert was verified by hybridization to viral RNA fractionated on a 0.9% agarose gel electrophoresis in presence of formaldehyde (Smiley, et al, Anal Biochem (1983) 131:365-372). Stringent hybridization conditions were used: prehybridizations and hybridizations were overnight at 42°C, and 50% formamide was used in hybridizations. Washing was at 65°C first with 2xSSC, 0.1% SDS and then with 0.2xSSC and 0.1% SDS.

In the foregoing verification, RNA from uninfected cells was used as negative control. The absence of exogenous viral sequences in the genome of the cells was verified by failure of cellular DNA digested with BamHI and EcoRI to bind to pDT28 probe in Southern blot analysis. The RNA from infected cells after 24 hrs of infection at a multiplicity greater than 1, and from the pellet of virus after complete cell lysis were used as positives. No hybridization was detected with the RNA from the uninfected cells, but the inserts hybridized to an approximately 13 kb band of the RNA isolated from the infected cells or from the pellet of virus.

The plasmid pDT28, which had been verified to contain a PstI insert which binds to the viral RNA, was used to probe the cDNA library for additional clones, and the entire sequence was recovered by "walking" techniques. In this way, eight additional plasmids were recovered which span the entire 12.5 kb genome of the virus. The positions of the overlapping inserts are shown in Figure 1. As shown in Figure 1, the pDT28 clone occupies a roughly central portion of the genome. The 8 additional plasmids recovered from the cDNA library in a manner analogous to that described above, but using the appropriate overlapping sequence-containing clone as probe, were grown in E. coli, and the plasmid DNA isolated. The inserts were sequenced, and verified to contain overlapping portions. The results of this sequencing are shown in Figure 2, which provides the entire genomic RNA sequence ascertained from the inserts.

The orientation shown in Figure 2 was determined by subcloning pDT28 into M13 into both orientations, labeling the resultant phage, and using the labeled phage as a probe against RNA known to be of positive polarity. This was done by spot hybridization on nitrocellulose filters using uninfected cell RNA, infected cell RNA, and template viral RNA. The infected cell RNA and template RNA should be of positive polarity. Therefore, the M13 orientation hybridizing to infected cell RNA and viral RNA contains a negative sense strand, and from this information, the 5' to 3' sequence of inserts from pCT63 to pCT185 could be deduced.

This conclusion was confirmed by analysis of the sequence of pCT63, which indicates its capability to form the expected hairpin structure at the 5' end, and by the absence of additional clones in the cDNA library having additional 5' sequences to that of pCT63.

### D.3. Expression of Sequences Encoding βGal-BDV Fusions in E. coli

Twelve portions of the BDV genome were obtained as follows: (1) the entire cDNA sequences per se, (2) products of restriction cleavage (with PstI or BamHI or both) of the foregoing cDNAs, and (3) a ligated sequence obtained by ligating the pCT185 cDNA with a fragment of another. (See the table below.) These portions were used to encode the BDV portions of the fusion proteins. These eleven BDV protein encoding sequences were cloned into one of or a mixture of pUR290, pUR291, and pUR292, which contain restriction sites, e.g., BamHI and PstI sites in all three possible reading frames with the β-gal codons, so as to encode fusion proteins at the C-terminal portion of the β-galactosidase protein (Ruther, U., et al, Embo J (1980) 2:1791-1794). Since all three possible reading frames are provided for the restriction sites used, the correct reading frame in at least one of the vectors for the fusion protein is assured. Table 1 summarizes the vectors prepared and the BDV sequence contained in each. Nucleotide numbers are as indicated in Figure 2.

**Table 1**

| Name | pUR Parent | BDV Insert Derived from | BDV Nucleotides Contained in pUBVD Vectors (Numbers as in Fig. 2) |
|---|---|---|---|
| pUBVD1 | pUR290 | pCT63 | 1397-2607 |
| pUBVD2 | pool | pCT36 | 2037-2574 |
| pUBVD4 | pUR292 | pCT183 | 2955-4560 |
| pUBVD5 | pool | pDT28 | 5650-6450 |
| pUBVD6 | pUR290 | pCT174 | 7225-10718 |
| pUBVD7 | pool | pCT174 | ∼9500-10811 |
| pUBVD8 | pUR292 | pDT65 | 10442-10811 |
| pUBVD9 | pUR292 | pDT65 + pCT185 | 10442-12470 |
| pUBVD10 | pUR290 | pCT185 | 11030-12457 |
| pUBVD11 | pUR290 | pCT185 | 11405-12457 |
| pUBVD12 | pUR291 | pCT63 | 597-1397 |
| pUBVD13 | pUR290 | pDT28 + pDT17 | ∼6000-∼7800 |

Each of the twelve cDNA sequences was mixed with T4 ligase in the presence of PstI-digested mixtures of pUR290, 291, and 292 (or of one of these if the correct reading frame was deduced) and the ligation mixture transformed into E. coli strain D1210 (Lacl⁻ mutant of HB101) to Amp^{R}. Successful transformants were confirmed by hybridization with labeled insert, and isolated plasmid DNA was analyzed by restriction analysis to confirm correct orientation. Expression was induced in successful transformants containing correctly oriented inserts by treating with IPTG (1 mM) on L-broth medium containing 40 µg/ml ampicillin. Three hours after induction, the cells were harvested, and lysed by sonication. The fusion proteins were produced as inclusion bodies, and the inclusion bodies were harvested by the method of Klempnauer, et al, Cell (1983) 33:345-355, and stored at -20°C suspended in 10 mM Tris (pH 8.0), 1 mM EDTA. Approximately 10-30 mg inclusion body proteins were obtained per ml of culture.

### D.4. Characterization of the Fusion Proteins

The fusion proteins were characterized as to their antigenic properties both in insoluble and solubilized forms.

Inclusion body proteins solubilized in 1% SDS or 7 M urea followed by dialysis to a final concentration of 1 mg/ml are unreactive with sera from infected calves or from rabbits infected with purified virus.

Preparation of Antisera. Both solubilized and unsolubilized inclusion bodies were injected into rabbits using peri-lymph nodal immunizations with 500 µg protein emulsified with Freund's complete adjuvant, with boosting every 4 weeks (IM injection of 500 µg emulsified in adjuvant) and bled 10 days after boost. Control antisera were prepared from infected calves or from rabbits injected with purified virus. The antisera were tested for immunoactivity by ELISA and immunofluorescence, and by Western blot and immunoprecipitation.

Western blot and immunoprecipitation yield complementary information with respect to reactivity. In immunoprecipitation, the native protein mixture is reacted with the test serum and the immunoprecipitate subjected to SDS-PAGE. Therefore, immunoprecipitation assesses immunoreactivity with the native protein.

However, in the Western SDS blot procedure, PAGE is performed before the antisera are tested for precipitation with the proteins on the gel. Therefore, Western blot assesses reactivity with denatured protein.

The results of these procedures are given below.

Results. The control antisera were immunoreactive with respect to proteins extracted from the virus pellet produced on BEKI cells, and showed immunoprecipitation with the 76 kD protein presumed to be the major antigenic component, as well as minor components presumed to be, at least in part, virion proteins having molecular weights of 36, 43, 47, 51 and 56 kD. No immunoprecipitation occurred when the control antisera were tested on Western blot. Control antisera against infection thus react with antigens in the native protein, but not after denaturation.

Immunoprecipitation and Western Blot. Most of the antisera formed in response to the fusion proteins were negative both in assay by immunoprecipitation and, like the control antisera, on Western blot.

However, there were exceptions. The antiserum generated by fusion protein 7 immunoprecipitates the 36 kD protein from BEKl-grown virus and reacts by Western blot to the 76 kD and 51 kD bands. Antiserum from fusion 5 immunoprecipitates 3 sizes of proteins: 64, 98, and 105 kD, sizes not precipitated by control antisera. Antiserum from fusion 9 precipitates a 58 kD band, also not precipitated by the control antisera. The significance of MW of the materials is not clear since it is not clear which, if any, of these proteins represent glycosylated materials with corresponding alterations in molecular weight.

ELISA (carried out according to the procedure of Bartlett, et al, in Protides of the Biological Fluids, H. Peeters, ed., Pergamon Press, Oxford, 1976, 24:767-770) used partially purified virus as antigen. Only the antiserum prepared against fusion protein 7 was positive at a 1:40 titer; serum prepared against fusion proteins 5 and 11 had titers of 1:4 and 1:8, respectively. Nonimmune sera were negative.

Immunofluorescence was conducted using labeled live or fixed infected cells. The antiserum prepared against fusion protein 11 was slightly positive in immunoreactivity with live cells; on cells fixed with methanol, acetone, or formaldehyde, serum prepared from fusion protein 7 gave the same strong response as control antisera from the infected animals, whereas antisera 5 and 3 were weakly positive against proteins extracted from the virus pellet produced on BEKI cells.

## Claims

1. A nucleotide sequence encoding a bovine diarrhea related polypeptide which is a region of the bovine diarrhea virus genomic sequence shown in Figure 2, or a combination of regions of that sequence.

2. A nucleotide sequence encoding a viral polypeptide substantially identical with that encoded by the bovine diarrhea virus genomic sequence shown in Figure 2.

3. A recombinant expression system capable, in a compatible host cell, of effecting the production of a bovine diarrhea virus related polypeptide which system comprises a DNA sequence of claim 1 being operably linked to a control sequence compatible with said host.

4. The system of claim 3 which further includes upstream of said DNA sequence, and in reading frame therewith, a fused nucleotide sequence encoding a host protein or portion thereof.

5. The system of claim 4 wherein the fusion DNA sequence encodes an N-terminal portion of β-galactosidase.

6. A recombinant vector which comprises the expression system of claim 3.

7. Recombinant host cells transformed with the vector of claim 6 or with a vector comprising the system of claim 4 or 5.

8. Polypeptide substantially identical with the entirety of the amino acid sequence as represented in Figure 2, or with a region or combination of regions thereof.

9. Polypeptide according to claim 8, which is further fused to a host protein or portion thereof.

10. A vaccine effective against bovine diarrhea virus which comprises the polypeptide of claim 8, and pharmaceutically acceptable excipients.

11. A vaccine according to claim 10, further containing an immunogenic particle, which particle comprises a polypeptide having an amino acid sequence capable of forming a particle when said sequence is produced in a eucaryotic host.

12. The vaccine of claim 11 wherein the particle forming amino acid sequence is derived from hepatitis B virus.

13. The vaccine of claim 12 wherein the particle forming amino acid sequence is derived from HBsAg.

14. A method for preparing a polypeptide according to claim 8, which comprises culturing the cells of claim 7 and recovering the recombinant polypeptide.

15. A method for preparing an anti-bovine diarrhea virus vaccine which comprises the method of claim 14, and further adding pharmaceutically acceptable excipients.

16. Use of a polypeptide according to any of claims 8 and 9 for the preparation of tests for the immunological detection of fusion proteins of the bovine diarrhea virus.

17. Use of a nucleotide sequence according to claim 1 for the construction of oligomeric sequences useful as diagnostic probes.

18. Use of a nucleotide sequence according to claim 1 for the preparation of a vaccine comprising as carrier a live viral vector permitting expression of a desired bovine diarrhea virus antigen along with a carrier's protein in infected cells.

## Patentansprüche

1. Nukleotidsequenz, die den Code eines mit der Rinderdiarrhoe zusammenhängenden Polypeptids enthält, und die ein Bereich der in der Figur 2 wiedergegebenen Genomsequenz des Rinderdiarrhoevirus, oder eine Kombination von Bereichen dieser Genomsequenz ist.

2. Nukleotidsequenz, die den Code eines Viruspolypeptids enthält, das im wesentlichen mit dem Viruspolypeptid identisch ist, dessen Code der in der Figur 2 wiedergegebenen Genomsequenz des Rinderdiarrhoevirus entspricht.

3. Rekombinierendes Expressionssystem, das in der Lage ist, in einer kompatiblen Wirtszelle die Produktion eines mit dem Rinderdiarrhoevirus zusammenhängenden Polypeptids durchzuführen, wobei dieses System eine DNS-Sequenz gemäß Anspruch 1 umfaßt, die mit einer mit diesem Wirt kompatiblen Steuersequenz funktionsfähig verbunden ist.

4. System gemäß Anspruch 3, das außerdem vor dieser DNS-Sequenz, und in dem zugehörigen Leserahmen, eine verschmolzene Nukleotidsequenz enthält, die den Code eines Wirtsproteins oder eines Abschnitts davon enthält.

5. System gemäß Anspruch 4, bei dem die Sequenz der Verschmelzungs-DNS den Code eines N-Endabschnitts von β-Galaktosidase enthält.

6. Rekombinationsvektor, der das Darstellungssystem von Anspruch 3 enthält.

7. Rekombinationswirtszellen, die mit dem Vektor von Anspruch 6, oder mit einem Vektor, der das System von Anspruch 4 oder 5 enthält, umgewandelt wurden.

8. Polypeptid, das im wesentlichen mit der Gesamtheit der Aminosäuresequenz, wie in der Figur 2 dargestellt, oder mit einem Bereich oder einer Kombination von Bereichen davon identisch ist.

9. Polypeptid gemäß Anspruch 8, das außerdem mit einem Wirtsprotein oder einem Abschnitt davon verschmolzen ist.

10. Impfstoff, der gegen den Rinderdiarrhoevirus wirksam ist, und der das Polypeptid des Anspruchs 8 und pharmazeutisch akzeptable Arzneiträger enthält.

11. Impfstoff gemäß Anspruch 10, der außerdem ein Immunität erzeugendes Partikel enthält, wobei dieses Partikel ein Polypeptid umfaßt, das eine Aminosäuresequenz aufweist, die in der Lage ist, ein Partikel zu bilden, wenn diese Sequenz in einem eucaryotischen Wirt produziert wird.

12. Impfstoff gemäß Anspruch 11, bei dem die Aminosäuresequenz, die das Partikel bildet, von einem Hepatitits B-Virus abgeleitet ist.

13. Impfstoff gemäß Anspruch 12, bei dem die Aminosäuresequenz, die das Partikel bildet, von HBsAg abgeleitet ist.

14. Methode zum Herstellen eines Polypeptids gemäß Anspruch 8, bei der die Zellen von Anspruch 7 gezüchtet werden, und das Rekombinationspolypeptid zurückgewonnen wird.

15. Methode zum Herstellen eines Impfstoffs gegen den Rinderdiarrhoevirus, die die Methode von Anspruch 14 umfaßt, und bei der außerdem pharmazeutisch akzeptable Arzneiträger hinzugegeben werden.

16. Verwendung eines Polypeptids gemäß irgendeinem der Ansprüche 8 oder 9 zur Entwicklung von Tests zum immunologischen Nachweis von Verschmelzungsproteinen des Rinderdiarrhoevirus.

17. Verwendung einer Nukleotidsequenz gemäß Anspruch 1 zum Aufbau von oligomeren Sequenzen, die als Diagnosesonden nützlich sind.

18. Verwendung einer Nukleotidsequenz gemäß Anspruch 1 zur Herstellung eines Impfstoffs, der als Träger einen lebenden Virusvektor aufweist, der in Verbindung mit dem Protein eines Trägers die Darstellung eines gewünschten Rinderdiarrhoevirus-Antigens in infizierten Zellen ermöglicht.

## Revendications

1. Séquence de nucléotides codant pour un polypeptide apparenté à la diarrhée bovine qui est une région de la séquence génomique du virus de la diarrhée bovine représentée dans la figure 2 ou une combinaison de régions de cette séquence.

2. Séquence de nucléotides codant pour un polypetide viral sensiblement identique à celui codé par la séquence génomique du virus de la diarrhée bovine représentée dans la figure 2.

3. Système d'expression recombinant susceptible d'effectuer, dans une cellule-hôte compatible, la production d'un polypeptide apparenté au virus de la diarrhée bovine, ce système comprenant une séquence d'ADN selon la revendication 1 qui est fonctionnellement liée à une séquence de contrôle compatible avec l'hôte précité.

4. Système selon la revendication 3 qui comprend par ailleurs, en amont de la séquence d'ADN précitée et en phase de lecture avec elle, une séquence de nucléotides fusionnée codant pour une protéine-hôte ou une partie de celle-ci.

5. Système selon la revendication 4, dans lequel la séquence d'ADN de la fusion code pour une partie N-terminale de bêta-galactosidase.

6. Vecteur recombinant qui comprend le système d'expression de la revendication 3.

7. Cellules-hôtes recombinantes transformées par le vecteur de la revendication 6 ou par un vecteur comprenant le système de la revendication 4 ou 5.

8. Polypetide sensiblement identique à la totalité de la séquence d'acides aminés telle que représentée dans la figure 2 ou à une région ou une combinaison de régions de celle-ci.

9. Polypeptide selon la revendication 8, qui est par ailleurs fusionné avec une protéine-hôte ou une partie de celle-ci.

10. Vaccin efficace contre le virus de la diarrhée bovine qui comprend le polypeptide de la revendication 8 et des excipients pharmaceutiquement acceptables.

11. Vaccin selon la revendication 10, contenant par ailleurs une particule immunogénique qui comprend un polypeptide ayant une séquence d'acides aminés susceptible de former une particule lorsque la séquence précitée est produite dans un hôte eucaryotique.

12. Vaccin selon la revendication 11, dans lequel la séquence d'acides aminés formant la particule est dérivée du virus de l'hépatite B.

13. Vaccin selon la revendication 12, dans lequel la séquence d'acides aminés formant la particule est dérivée du HBsAg.

14. Procédé de préparation d'un polypeptide selon la revendication 8, qui consiste à cultiver les cellules de la revendication 7 et à récupérer le polypeptide recombinant.

15. Procédé de préparation d'un vaccin contre le virus de la diarrhée bovine qui comprend le procédé de la revendication 14 en ajoutant par ailleurs des excipients pharmaceutiquement acceptables.

16. Emploi d'un polypeptide selon l'une quelconque des revendications 8 et 9 pour la préparation de tests permettant la détection immunologique de protéines de fusion du virus de la diarrhée bovine.

17. Emploi d'une séquence de nucléotides selon la revendication 1 pour la construction de séquences oligomères susceptibles d'être utilisées comme sondes de diagnostic.

18. Emploi d'une séquence de nucléotides selon la revendication 1 pour la préparation d'un vaccin comprenant comme véhicule un vecteur viral vivant permettant l'expression d'un antigène souhaité du virus de la diarrhée bovine conjointement avec une protéine du véhicule dans les cellules infectées.
